# EUROPEAN PATENT APPLICATION

(11) **EP 4 696 363 A2**
(43) Date of publication of application: **18.02.2026**
(21) Application number: 25226932.9
(22) Date of filing: 31.03.2023
(51) Int. Cl.: A61N 1/04

(54) **ALTERNATING ELECTRIC FIELD WAVEFORM FOR ELECTROSENSATION REDUCTION**

(30) Priority: 31.03.2022 US 202263326098 P; 27.09.2022 US 202263410481 P; 30.03.2023 US 202318128937
(62) Divisional of application: 23719487.3
(71) Applicant: Novocure GmbH, 6340 Baar (CH)
(72) Inventor: GILADI, Moshe, 31905 Haifa (IL); AVIGDOR, Lilach, 31905 Haifa (IL)
(74) Representative: Carpmaels & Ransford LLP

(57) **Abstract**

An apparatus to treat a target region of a subject's body with an alternating electric field and being capable of: applying the alternating electric field to the target region of the subject's body at a first frequency while increasing an intensity of the alternating electric field from zero to a target intensity; and changing the frequency of the alternating electric field from the first frequency to a target frequency while maintaining the alternating electric field at the target intensity, wherein the target frequency and the target intensity have a known efficacy for treating the target region of the subject's body with the alternating electric field; and wherein the first frequency is greater than the target frequency.

## Description

### CROSS-REFERENCE

This application claims priority to U.S. Patent Application No. 18/128,937 filed March 30, 2023, U.S. Provisional Application No. 63/326,098, filed March 31, 2022, and U.S. Provisional Application No. 63/410,481, filed September 27, 2022, the entire contents of each of which are incorporated herein by reference.

### BACKGROUND

Tumor treating fields (TTFields) are low intensity alternating electric fields within the intermediate frequency range, which may be used to treat tumors as described in U.S. Patent No. 7,565,205. TTFields are induced non-invasively into a region of interest by transducers placed directly on a patient's body and applying AC voltages between the transducers. AC voltage is applied between a first pair of transducers for a first interval of time to generate an electric field with field lines generally running in the front-back direction. Then, AC voltage is applied at the same frequency between a second pair of transducers for a second interval of time to generate an electric field with field lines generally running in the right-left direction. The system then repeats this two-step sequence throughout the treatment.

### BRIEF DESCRIPTION OF THE DRAWINGS

FIG. 1A depicts an example method of treating a target region of a subject's body.
FIG. 1B depicts an example method of treating a target region of a subject's body.
FIG. 2 depicts an example system having four transducers.
FIG. 3 depicts an example apparatus to control an alternating electric field.
FIGS. 4-6 depict example waveforms of an alternating electric field.
FIG. 7 depicts another example method of treating a target region of a subject's body.
FIG. 8 depicts an example method of delivering an alternating electric field to a subject's body.
FIG. 9 depicts an example method of delivering an alternating electric field to a subject's body as temperature rises.
FIG. 10 depicts an example cytotoxicity analysis showing the effects of the field intensity.
FIGS. 11A-11C depict an experimental stimulation of a sciatic rat nerve bundle with alternating electric fields.

### DESCRIPTION OF EMBODIMENTS

This application describes exemplary systems and methods for delivering alternating electric fields to a subject's body for treatment of the subject's body. In particular, the present disclosure relates to systems and methods for applying an alternating electric field according to a waveform that reduces electrosensation in a subject.

When treating a subject using alternating electric fields, higher intensities (e.g., amplitudes) may be strongly associated with higher efficacy of treatment. However, at higher intensity (e.g., amplitude) of the alternating electric field, and/or lower frequency of the alternating electric field (e.g., in the vicinity of 100 kHz), some subjects experience an electrosensation effect, particularly at the onset of the alternating electric field.

The electrosensation may originate from interactions between the alternating electric fields and nerve cells that are positioned near or adjacent to the transducer arrays inducing the alternating electric fields. For example, an AC current at a given frequency may be coupled into a subject's body through a pair of identical electrodes that are positioned on the subject's skin for applying the alternating electric field. When the current density (e.g., the current through any given electrode divided by the area of that electrode) is below a threshold value (which may vary from person to person), electrosensation may not occur. When the current density rises above that threshold, however, electrosensation can start to occur and can become more noticeable when the current density is increased further. This electrosensation could be, for example, a vibratory sensation, paresthesia, and/or a twitching or contraction sensation of muscle fibers. These sensations may discourage some subjects from continuing their treatment using alternating electric fields.

The inventors realized these problems and discovered methods and systems for reducing or eliminating electrosensation while a subject is treated with alternating electric fields. In particular, the inventors realized that electrosensation occurs in response to current densities that are present during onset of a duty cycle of the alternating electric field. The present disclosure is directed to systems and methods for avoiding electrosensation at the onset of the duty cycle altogether by applying the alternating electric field at a higher frequency during the dimming up period. The alternating electric field is applied to the subject's body using a voltage generated according to an AC voltage waveform. In a given duty cycle, the AC voltage waveform has a higher frequency during the dimming up period, then switches to an optimal frequency for treating the subject's body at or near the time when the electric field reaches peak intensity. This hybrid frequency AC voltage waveform helps avoid electrosensation that might otherwise occur if the optimal frequency for treating the subject's body were used during the entire duty cycle.

The inventors discovered systems and methods for minimizing electrosensation at the onset of the duty cycle by applying the alternating electric field at a lower intensity and a higher frequency during the dimming up period. The alternating electric field may be applied to the subject's body using a voltage generated according to an AC voltage waveform. During the dimming up period of the duty cycle, the AC voltage waveform initially has a lower intensity than a target intensity and a higher frequency than a target frequency. As the dimming up period proceeds, the intensity is increased to the target intensity, and the frequency is decreased to the target frequency. This AC voltage waveform may help to avoid electrosensation that might otherwise occur if the target intensity and the target target frequency for treating the subject's body were used during the entire duty cycle.

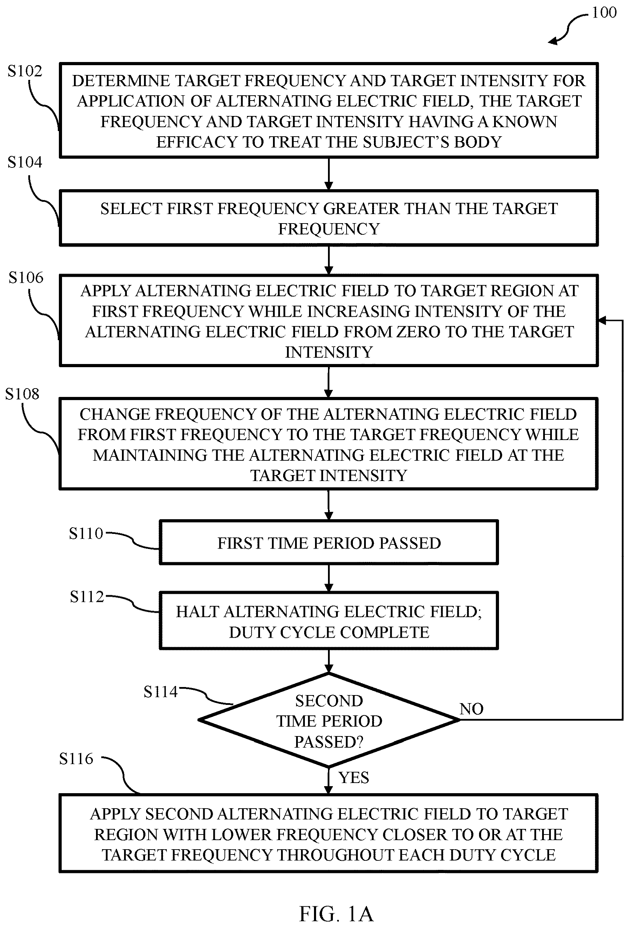

FIG. 1A depicts an example method 100 to treat a target region of a subject's body with an alternating electric field. The method 100 may include, at step S102, determining a target frequency and a target intensity for application of an alternating electric field. In an example, the target region may include at least one of cancer, a tumor, an endothelial or epithelial tight junction (e.g., such as a blood-brain barrier (BBB) or intestinal wall tight junction), or any tissues or cells of the subject. In an example, the target region may be determined by the treating of viral infections, bacterial infections, neurodegenerative diseases, and/or autoimmune diseases. In certain embodiments, the subject can experience electrosensation when an alternating electric field with the target intensity and the target frequency is applied.

The term "target frequency" used herein may refer to a clinically approved frequency that can provide a known efficacy for the desired treatment of a subject using an alternating electric field applied to the subject at the target frequency. For example, the target frequency can be a clinically approved frequency that provides a proven efficacy against tumor cell proliferation or for opening an endothelial or epithelial tight junction. For example, the target frequency may be determined from in vitro assay. The term "target frequency" may also refer to a frequency that is more effective for the intended treatment than a first frequency. In general, the target frequency is lower than the first frequency. The target frequency may be in a target frequency range corresponding to a particular target cell, tissue, and/or treatment.

In non-limiting embodiments, the target frequency may be approximately 50 kHz to approximately 10 MHz, approximately 50 kHz to approximately 1 MHz, approximately 50 kHz to approximately 500 kHz, or approximately 100 kHz to approximately 400 kHz. As an example, if the target region is a glioblastoma (GBM), the target frequency may be approximately 100 kHz to approximately 300 kHz for treating the GBM (e.g., 50 kHz - 350 kHz, or 150 kHz - 250 kHz, or 200 kHz). For example, if the treatment goal is to reduce tumor cell proliferation, the target frequency may be approximately 100 kHz to approximately 400 kHz (e.g., 150 kHz - 200 kHz, or 140 kHz - 220 kHz). As another example, if the target region is an epithelial or endothelial tight junction, the target frequency may be approximately 50 kHz to approximately 190 kHz for opening the epithelial or endothelial tight junction (e.g., 75 kHz - 190 kHz, or 100 kHz, or 150 kHz). As another example, if the treatment goal is to change cancer cell membrane permeability (e.g., increase cancer cell membrane permeability), the target frequency may be approximately 100 kHz to approximately 500 kHz (e.g., 50 kHz - 550 kHz, or 150 kHz - 350 kHz, or 250 kHz - 400 kHz). For example, if the treatment goal is to treat non-small cell lung cancer (NSCLC), the target frequency may be approximately 100 kHz to approximately 200 kHz (e.g., 150 kHz). For example, if the target region/tissue is pancreatic tissue/cancer, the target frequency may be approximately 100 kHz to approximately 200 kHz (e.g., 150 kHz). For example, if the target region/tissue is ovarian tissue/cancer, the target frequency may be approximately 150 kHz to approximately 250 kHz (e.g., 200 kHz). For example, if the target region/tissue is gastric tissue/cancer, the target frequency may be approximately 100 kHz to approximately 200 kHz (e.g., 150 kHz). Although this disclosure describes and illustrates particular frequency ranges, this disclosure contemplates any target frequency range configured to produce a proven efficacy for treating a subject.

In some embodiments, the term "target intensity" may refer to a clinically approved intensity that can provide a known efficacy for the desired treatment of a subject using an alternating electric field applied to the subject at the target intensity. For example, the target intensity can be a clinically approved intensity that provides a proven efficacy against tumor cell proliferation or for opening an epithelial or endothelial tight junction. For example, the target intensity may be determined from in vitro assay.

In some embodiments, the term "target intensity" may refer to an intensity that is more effective for the intended treatment than a first intensity applied to a subject. In general, the target intensity is higher than the first intensity. In certain embodiments, the target intensity may be in a target intensity range corresponding to a particular target cell, tissue, and/or treatment.

In some embodiments, the term "target intensity" may refer to an intensity based on a temperature related to the alternating electric field applied to the subject's body. For example, the target intensity may be based on a temperature of the subject's body measured at a location on the subject's body that receives the alternating electric field. For example, the target intensity may be based on a temperature of an electrode that is providing the alternating electric field to the subject's body. For example, the target intensity may be the maximum intensity that can be achieved without the subject's body and/or an electrode applying the alternating electric field exceeding a temperature threshold between a range (e.g., between approximately 37° C and approximately 43° C).

In some embodiments, the term "target intensity" may refer to a combination of two or more of the above exemplary target intensities, in the aggregate and/or in the alternative.

In non-limiting embodiments, the target intensity may be approximately 0.5 V/cm to approximately 10 V/cm, or approximately 0.1 V/cm to approximately 20 V/cm. For example, if the target region is a GBM, the target intensity may be approximately 1 V/cm to approximately 5 V/cm for treating the GBM (e.g., 1 V/cm - 3 V/cm). For example, if the treatment goal is to reduce tumor cell proliferation, the target intensity may be approximately 1 V/cm to approximately 5 V/cm (e.g., 1 V/cm - 3 V/cm). For example, if the target region is a BBB, the target intensity may be approximately 1 V/cm to approximately 5 V/cm (e.g., 1 V/cm - 3 V/cm) for opening the BBB. For example, if the treatment goal is to change cell membrane permeability (e.g., increase cell membrane permeability), the target intensity may be approximately 1 V/cm to approximately 5 V/cm (e.g., 1 V/cm - 3 V/cm).

Although this disclosure describes and illustrates particular frequency ranges and intensity ranges, this disclosure contemplates any target frequency range and any target intensity range having a known efficacy for treating a subject. In non-limiting embodiments, the target frequency and/or the target intensity can vary depending on the target region.

At step S104, the method 100 may include selecting a first frequency to reduce electrosensation in the subject's body. An alternating electric field that begins at the first frequency is able to reduce electrosensation of the subject compared to an alternating electric field that is applied at the target frequency while the intensity is ramped up to the target intensity. In one example, the first frequency may be selected by decreasing a frequency level of the alternating electric field stepwise from an initial frequency until the subject experiences the electrosensation, as indicated by the response of the subject. In one example, the first frequency may be selected by decreasing a frequency level of the alternating electric field stepwise from an initial frequency until the subject experiences the electrosensation, as indicated by an objective measure, such as detection of nerve stimulation.

The first frequency is greater than the target frequency. For example, the first frequency may be at least approximately 10% higher than the target frequency (e.g., at least approximately 20% higher, 30% higher, 50% higher, 75% higher, 100% higher, 150% higher, 200% higher, or 300% higher). As another example, the first frequency may be at least approximately 10 kHz higher than the target frequency (e.g., at least approximately 20 kHz higher, 30 kHz higher, 50 kHz higher, 75 kHz higher, 100 kHz higher, 150 kHz higher, 200 kHz higher, or 300 kHz higher). In an example, the first frequency may be between approximately 100 kHz and approximately 400 kHz, between approximately 200 kHz and approximately 600 kHz, between approximately 300 kHz and approximately 800 kHz, or between approximately 50 kHz and approximately 350 kHz. In an example, the first frequency may be as high as 900 kHz, 1 MHz, 10 MHz, or higher. The first frequency may depend on the current being applied to the transducer pairs. In general, a subject feels a greater degree of electrosensation at higher intensities. Accordingly, in some embodiments, a higher first frequency may be used when higher intensities (e.g.. higher currents) are applied to compensate for the greater degree of electrosensation at higher intensities. For example, in an embodiment where a 2 Amp current is applied, the first frequency may be between approximately 300 kHz and 400 kHz. As another example, in an embodiment where a 4 Amp current is applied, the first frequency may be between approximately 500 kHz and 600 kHz. In certain embodiments, the first frequency may be pre-determined based on certain criteria, such as the type of treatment, body part to which the transducers will be applied on the subject, and/or other information on the subject.

At step S106, the method 100 includes applying an alternating electric field to the target region of the subject's body at the first frequency while increasing an intensity of the alternating electric field from zero to the target intensity. This represents the beginning of a duty cycle of the alternating electric field. The duty cycle of the alternating electric field may begin with applying the alternating electric field at the first frequency while ramping up (or "dimming up") the intensity of the alternating electric field.

At step S108, the method 100 includes changing the frequency of the alternating electric field from the first frequency to the target frequency while maintaining the alternating electric field at the target intensity. In particular, the frequency of the alternating electric field decreases from the first frequency to the target frequency that is optimal for treatment of the subject's body. In an example, the frequency change may be immediate or may take less than approximately 200 ms, less than approximately 100 ms, less than approximately 50 ms, less than approximately 25 ms, less than approximately 10 ms, less than approximately 5 ms, less than approximately 1 ms, or less than approximately 0.1 ms to occur. In another example, the frequency change may be gradual (e.g., taking at least approximately 0.1 ms, at least approximately 1 ms, at least approximately 5 ms, at least approximately 10 ms, at least approximately 25 ms, at least approximately 50 ms, at least approximately 100 ms, or at least approximately 200 ms to occur and/or taking place in a stepwise manner). The change in frequency may take place immediately when or shortly after the alternating electric field reaches the target intensity.

The alternating electric field may continue at the target frequency until, at step S110, a first period of time has passed. The first period of time may be less than approximately 3 seconds (e.g., less than approximately 2 seconds, or less than approximately 1 second).

After the first period of time (S110), the method 100 may include, at step S112, halting application of the alternating electric field to complete a first duty cycle of the alternating electric field. The alternating electric field may be configured to reduce electrosensation of the subject compared to when an alternating electric field is applied at the target frequency for its entire duty cycle.

At step S114, the method 100 may include determining whether a second period of time has passed. The second period of time may be from approximately 12 hours to approximately 4 weeks (e.g., between approximately 48 hours and 3 weeks, or between approximately 1 week and 2 weeks). If the second period of time has not passed, the method 100 may repeat steps S106 to S114. That is, the method 100 may include applying one or more subsequent duty cycles of the alternating electric field of steps S106 to S 112. In certain embodiments, the method 100 may include alternately applying duty cycles of the alternating electric field via a first pair of transducer arrays and via a second pair of transducer arrays.

Once the second period of time (S114) has passed, the method 100 may include, at step S116, switching from applying the alternating electric field to the target region of the subject's body to applying a second alternating electric field to the target region of the subject's body with a second frequency that is lower than the first frequency and closer to or at the target frequency throughout each duty cycle. After the second period of time (S114), the subject's body may be desensitized to the current density at the electrode / skin interface such that the subject will no longer experience electrosensation during the dimming up period. As such, the second alternating electric field having the lower (or target) frequency throughout the entire duty cycle may be well tolerated by the subject and used to deliver higher efficacy of treatment.

In some embodiments, the alternating electric field can be cyclically applied to the target region. Cycling treatment involves the application of the alternating electric field for a period of time, followed by a rest for a period of time, and repeating this sequential administration. Cycling treatment may reduce or avoid electrosensation and/or may improve the efficacy of the treatment. In non-limiting embodiments, the number of cycles can be from approximately 1 to approximately 50, or from approximately 1 to approximately 24. In one example, the rest time can be from approximately ten seconds to approximately a week. In certain embodiments, the treatment stops when the subject experiences electrosensation and/or is intolerable to the adverse effects associated with the alternating electric field.

The subject's body may not experience electrosensation when the second alternating electric field is applied. In one example, the second alternating electric field can have a second frequency closer to or at the target frequency than the first frequency. In one example, the second alternating electric field can have a second intensity closer to or at the target intensity than the first intensity. In one example, the second alternating electric field can have a second frequency and a second intensity, which are the target frequency and the target intensity. In non-limiting embodiments, the first alternating electric field may be continuously switched to the second alternating electric field without turning off the system/transducer for a wait time. In an example, an alternating electric field may be applied, with or without pausing, while the parameters of the alternating electric field are changed between a first set and a second set. In an example, the alternating electric field with the first frequency and the first intensity can be ceased, and a second alternating electric field can be applied to the target region of the subject's body. "Ceased" may refer to the stoppage of the alternating electric field so that no alternating electric field is applied or a change in the parameters of the alternating electric fields so that, in effect, a different alternating electric field may then be applied.

It should be noted that the target frequency, target intensity, first frequency, first period of time, and second period of time may be specifically tailored to the individual subject receiving treatment, based on the target region and/or the presence or amount of electrosensation felt by the subject.

In one example, the alternating electric field can be applied to the target region in a single dose or divided doses. For example, the alternating electric field can be applied to the target region once a day, twice a day, once a week, twice a week, three times a week, four times a week, five times a week, six times a week, once every two weeks, once a month, twice a month, once every other month or once every third month.

During the treatment dose, the patient may take breaks within the treatment and still be considered dose. For example, a patient may receive the alternating electric field treatment approximately 80% of the time and take breaks. In one example, for opening a BBB, a patient may wear or equip the disclosed systems/devices for approximately three days (e.g., approximately 72 hours) to open the BBB but can have breaks during the treatment time. For tumors, the device may be used for extended periods of time (indefinitely), again with breaks for showering, swimming, changing the arrays, etc., with the goal being 80% of the treatment time the arrays are on. As an example, once a month may be useful for a BBB application, if the "once a month" is at least 72 hours, but for tumors, longer stretches of using the device may be required.

In an example, the alternating electric field can be applied to the target region for a predetermined interval of time. For example, the interval of time may be based on how long a typical subject may experience electrosensation at the target frequency and/or target intensity. For example, the interval of time may be based on how long the subject experiences electrosensation at the target frequency and/or target intensity. Such an interval of time may be subjective based on the subject.

In one example, the predetermined interval of time (e.g., duration of the alternating electric field treatment) can be between approximately one second and approximately 1 month. For example, the interval may be approximately 1 hour to approximately 12 hours, approximately 12 hours to approximately 24 hours, approximately 1 day to approximately 3 days, approximately 3 days to approximately 7 days, approximately 1 week to approximately 2 weeks, or approximately 1 week to approximately 3 weeks.

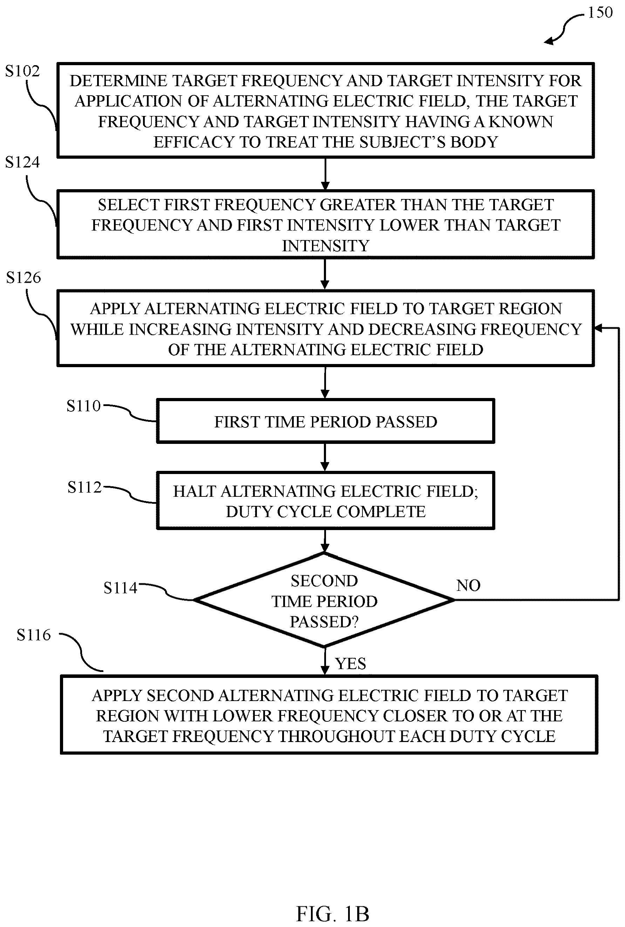

FIG. 1B depicts an example method 150 to treat a target region of a subject's body with an alternating electric field. The target region may be a similar target region as described above with regards to FIG. 1A. The method 150 may include step 102, as in FIG. 1A.

At step S124, the method 150 may include selecting a first frequency and a first intensity to reduce electrosensation in the subject's body. During a dimming period, the first frequency and the first intensity of the alternating electric field are adjusted to the target frequency and the target intensity, which may be able to reduce electrosensation of the subject when compared to initially applying the alternating electric field with the target frequency and/or the target intensity. The first frequency is greater than the target frequency as described above in step S104 of FIG. 1A.

The first intensity is lesser that the target intensity. For example, the first intensity may be at least approximately 10% lower than the target intensity (e.g., at least approximately 20% lower, 30% lower, 50% lower, 75% lower, 100% lower, 150% lower, 200% lower, or 300% lower). For example, the first intensity may be lower than the target intensity by at least 0.1 V/cm lower (e.g., at least approximately 0.2 V/cm, 0.3 V/cm, 0.4 V/cm, 0.5 V/cm, 0.6 V/cm, 0.7 V/cm, 0.8 V/cm, 0.9 V/cm, 1 V/cm, 1.5 V/cm, 2 V/cm, 2.5 V/cm, 3.0 V/cm, 3.5 V/cm, or 4.5 V/cm lower). In an example, the first intensity may be between approximately 0.1 V/cm and approximately 4.5 V/cm, between approximately 0.5 V/cm and approximately 3.0 V/cm, between approximately 0.1 V/cm and approximately 2.5 V/cm. In an example, the first intensity may be 0 V/cm, slightly above 0 V/cm, or approximately 0 V/cm.

At step S126, the method 150 includes applying an alternating electric field to the target region of the subject's body while increasing the intensity of the alternating electric field from the first intensity to the target intensity and decreasing the frequency of the electric field from the first frequency to the target frequency. This represents the beginning of a duty cycle of the alternating electric field. The duty cycle of the alternating electric field may begin with applying the alternating electric field at the first intensity and the first frequency while ramping up (or "dimming up") the intensity of the alternating electric field and simultaneously decreasing the frequency of the alternating electric field.

In an example, the frequency change may be immediate or may take less than approximately 200 ms, less than approximately 100 ms, less than approximately 50 ms, less than approximately 25 ms, less than approximately 10 ms, less than approximately 5 ms, less than approximately 1 ms, or less than approximately 0.1 ms to occur. In another example, the frequency change may be gradual (e.g., taking at least approximately 0.1 ms, at least approximately 1 ms, at least approximately 5 ms, at least approximately 10 ms, at least approximately 25 ms, at least approximately 50 ms, at least approximately 100 ms, or at least approximately 200 ms to occur and/or taking place in a stepwise manner).

The change in frequency may correspond to an equivalent change in intensity. Alternatively, the rate of increase of intensity and rate of decrease of frequency may differ. The rate of increase of intensity and the rate of decrease of frequency do not need to start and stop at the same time. In some embodiments, an overlap of increasing intensity and decreasing frequency may occur at the onset of treatment (e.g., the overlap may occur at least within, for example, the first 0.1 seconds of treatment).

In an example, the intensity change may be immediate or may take less than approximately 200 ms, less than approximately 100 ms, less than approximately 50 ms, less than approximately 25 ms, less than approximately 10 ms, less than approximately 5 ms, less than approximately 1 ms, or less than approximately 0.1 ms to occur. In another example, the intensity change may be gradual (e.g., taking at least approximately 0.1 ms, at least approximately 1 ms, at least approximately 5 ms, at least approximately 10 ms, at least approximately 25 ms, at least approximately 50 ms, at least approximately 100 ms, or at least approximately 200 ms to occur and/or taking place in a stepwise manner).

After step S126, the method 150 proceeds to steps S110, S112, S114, and S116 as in FIG. 1A. However, the "no" branch from step S114 goes to step S126 in FIG. 1B. As such, for FIG. 1B, the method 150 may include applying one or more subsequent duty cycles of the alternating electric field of steps S126, S110, and S112.

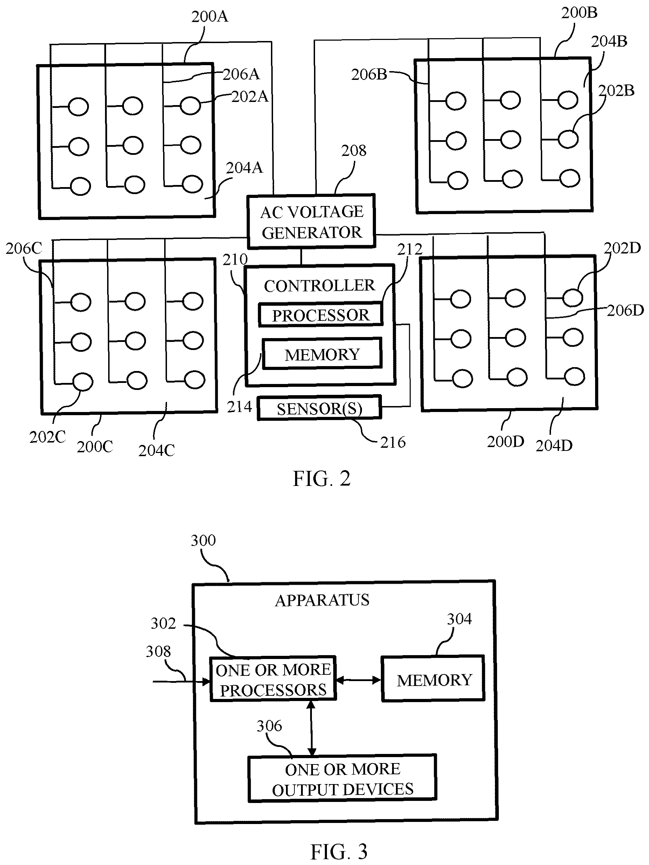

FIG. 2 depicts an example system having four transducers (or "transducer arrays") 200A-D. The system may be used for treating a target region of a subject's body with an alternating electric field. In an example, the target region may be in the subject's brain, and an alternating electric field may be delivered to the subject's body via a pair of transducer arrays positioned on a head of the subject's body. In another example, the target region may be in the subject's torso, and an alternating electric field may be delivered to the subject's body via a pair of transducer arrays positioned on at least one of a thorax, an abdomen, or one or both thighs of the subject's body. Other transducer array placements on the subject's body may be possible.

Each transducer 200A-D may include substantially flat electrode elements 202A-D positioned on a substrate 204A-D and electrically and physically connected (e.g., through conductive wiring 206A-D). The substrates 204A-D may include, for example, cloth, foam, flexible plastic, and/or conductive medical gel. Two transducers (e.g., 200A and 200D) may be a first pair of transducers configured to apply an alternating electric field to a target region of the subject's body. The other two transducers (e.g., 200B and 200C) may be a second pair of transducers configured to similarly apply an alternating electric field to the target region.

The transducers 200A-D may be coupled to a voltage generator 208 (i.e., AC voltage generator), and the system further includes a controller 210 communicatively coupled to the AC voltage generator 208. The controller 210 may include a computer having one or more processors 212 and memory 214 accessible by the one or more processors. The memory 214 may store instructions that when executed by the one or more processors control the voltage generator 208 to induce alternating electric fields between pairs of the transducers 200A-D according to one or more voltage waveforms and/or cause the computer to perform one or more methods disclosed herein. The controller 210 may monitor operations performed by the AC voltage generator 208 (e.g., via the processor(s) 212). One or more sensor(s) 216 may be coupled to the controller 210 for providing measurement values or other information to the controller 210.

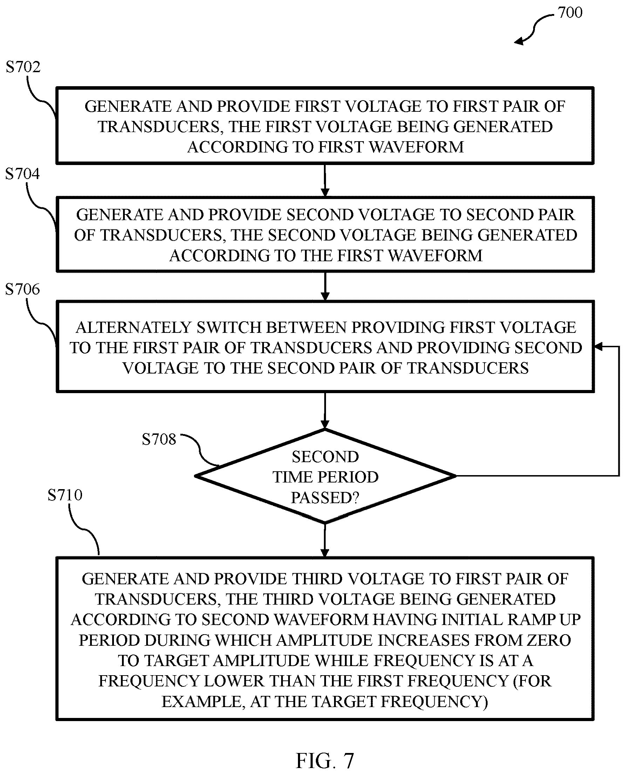

The voltage generator 208 may provide one or more voltages to the different pairs of transducers (e.g., 200A/D, 200B/C) for applying alternating electric fields to the subject's body. The controller 210 may instruct the voltage generator 208 to generate the one or more voltages according to one or more waveforms. For example, the method described below with reference to FIG. 7 may be implemented using the voltage generator 208 and controller 210.

In an example, the controller 210 may provide signals to the first pair of transducers (200A, 200D) for controlling a first alternating electric field according to a first waveform such that: the first alternating electric field begins at a first frequency; the first alternating electric field increases from zero intensity to a target intensity while remaining at the first frequency; and the first alternating electric field changes from the first frequency to a target frequency after reaching the target intensity, the target frequency being lower than the first frequency. Similarly, the controller 210 may provide signals to the second pair of transducers (200B, 200C) for controlling a second alternating electric field according to the same or similar waveform.

FIG. 3 depicts an example apparatus for use with the embodiments herein. As an example, the apparatus 300 may be a computer to implement certain techniques disclosed herein. As an example, the apparatus 300 may be a controller apparatus to apply alternating electric fields for the embodiments herein. For example, the apparatus 300 may be used as the controller 210 of FIG. 2. The apparatus 300 may include one or more processors 302, a memory 304, and one or more output devices 306. The memory 304 is accessible by the one or more processors 302 so that the one or more processors 302 can read information from and write information to the memory 304. The memory 304 may store instructions that when executed by the one or more processors 302 implement one or more embodiments of the present disclosure. For example, the apparatus 300 may include one or more processors and memory accessible by the one or more processors, where the memory stores instructions that when executed by the one or more processors, cause the apparatus 300 to perform operations to implement one or more embodiments of the present disclosure.

In one example, based on input 308, the one or more processors 302 may generate control signals to control the voltage generator to implement an embodiment of the invention. The input 308 may be user input, sensor input, or input from another computer in communication with the apparatus 300. The output devices 306 may provide the status of the operation of the invention, such as transducer selection, voltages being generated, and other operational information. The output devices 306 may provide visualization data.

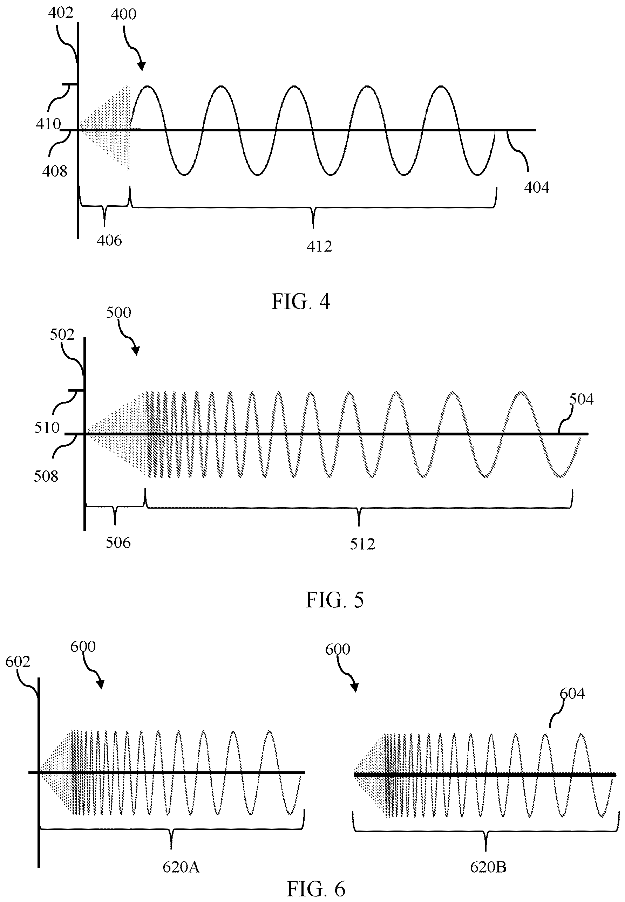

FIGS. 4-6 depict example waveforms (400, 500, 600) that may be used to control an alternating electric field and/or are representative of an alternating electric field in accordance with the present disclosure. The waveforms (400, 500, 600) show patterns of alternating electric fields during one or more duty cycles according to the method of FIG. 1. In an example, the waveforms (400, 500, 600) may represent an amplitude (402, 502, 602) of the intensity of the alternating electric field with respect to time (404, 504, 604), or an amplitude (402, 502, 602) of the voltage applied to a pair of transducers with respect to time (404, 504, 604).

As shown in FIGS. 4 and 5, the waveform (400, 500) may include an initial ramp up period (406, 506) during which the amplitude (402, 502) of the waveform (400, 500) increases from zero (408, 508) to a target amplitude (410, 510) while a frequency of the waveform (400, 500) is at a first frequency. The waveform (400, 500) also includes a sustained intensity period (412, 512) during which the frequency of the waveform (400, 500) changes to a target frequency that is lower than the first frequency while the amplitude (402, 502) of the waveform (400, 500) remains at the target amplitude (410, 510).

As shown in FIG. 4, the change in the frequency of the waveform 400 (e.g., alternating electric field) from the first frequency to the target frequency may be performed instantly (or nearly instantly) at one time during application of the alternating electric field. In an example, the change in the frequency of the waveform 400 from the first frequency to the target frequency may be performed in less than a period of 200 ms. As such, the waveform 400 may start at a higher frequency during the initial ramp up period 406 and then switch instantly (or near instantly) to a lower, optimal treatment frequency once a high enough amplitude 402 is reached.

As shown in FIG. 5, the change in the frequency of the waveform 500 (e.g., alternating electric field) from the first frequency to the target frequency may be performed gradually over a period of time during application of the alternating electric field (e.g., during the sustained intensity period 512). As such, the waveform 500 may start at a higher frequency during the initial ramp up period 506 and then switch gradually to a lower, optimal treatment frequency once a high enough amplitude 502 is reached. As shown in FIG. 5, the frequency gradually changes from the first frequency to the target frequency during the sustained intensity period 512. In some embodiments, the frequency gradually may change from the first frequency to the target frequency during the initial ramp up period 506 or during both the initial ramp up period 506 and the sustained intensity period 512.

An alternating electric field according to the present disclosure may be applied using a voltage generated according to an AC voltage waveform (e.g., 400, 500). As shown in FIGS. 4 and 5, the change in frequency from the first frequency to the target frequency may be performed at one or more times each corresponding to a zero crossing of the AC voltage waveform (e.g., 400, 500). A "zero crossing" may be any point in time (404, 504) where the amplitude (402, 502) is zero (408, 508). In one example, as shown in FIG. 4, the change in frequency may be performed at a single time corresponding to a zero crossing of the waveform 400. In another example, as shown in FIG. 5, the change in frequency may be performed in a stepwise manner with each shift happening at a different zero crossing of the waveform 500. Changing the frequency at one or more zero crossings provides a smooth transition of the electric field applied to the subject's body such that no shock or electrosensation occurs in the subject's body.

FIGS. 4 and 5 each represent a single duty cycle of an alternating electric field applied according to a waveform (e.g., "first waveform" as described above with reference to FIG. 2). FIG. 6 represents two duty cycles 620A and 620B of an alternating electric field. The duty cycles 620A and 620B may be for the same transducer or different transducers. As shown, the second duty cycle 620B follows a similar pattern of intensities (e.g., amplitudes 602) and frequencies as the first duty cycle 620A. The term "similar pattern" may refer to a general pattern in which the frequency changes to a lower frequency after the initial ramp up period (i.e., once the amplitude 602 reaches the target level). As such, the frequencies and amplitudes need not be exactly the same between the first duty cycle 620A and the second duty cycle 620B as long as they follow the same general pattern. In some embodiments, as shown in FIG. 6, the duty cycles 620A, 620B may be applied according to an identical waveform 600 (i.e., the first frequency, target frequency, and time(s) when the frequency is changed are the same in both duty cycles).

FIG. 7 depicts an example method 700 of treating a target region of a subject's body with an alternating electric field. The method 700 may be implemented using the voltage generator 208 and controller 210 of FIG. 2. The method 700 may include, at step S702, generating and providing a first voltage to a first pair of transducers, the first voltage being generated according to a first waveform (e.g., AC voltage waveform). The first waveform may be similar to waveforms shown in FIGS. 4-6. That is, the first waveform may include an initial ramp up period during which the amplitude increases from zero to a target amplitude while the frequency is at a first frequency, after which the frequency changes to a target frequency lower than the first frequency while the amplitude remains at the target amplitude.

At step S704, the method 700 may include generating and providing a second voltage to a second pair of transducers, the second voltage being generated according to the first waveform (e.g., AC voltage waveform). At step S706, the method 700 may include alternately switching between providing the first voltage to the first pair of transducers and providing the second voltage to the second pair of transducers. Step S706 may be repeated until a second time period has passed at step S708. After the second time period has passed, the method 700 proceeds to step S710. The second time period (S708) of FIG. 7 may be the same as the second time period (S114) of FIG. 1.

At step S710, the method 700 may include generating and providing a third voltage to the first pair of transducers, the third voltage being generated according to a second waveform (e.g., AC voltage waveform). The second waveform may include an initial ramp up period during which the amplitude of the second waveform increases from zero to a target amplitude while a frequency of the second waveform is at a frequency lower than the first frequency. The frequency lower than the first frequency may be equal to the target frequency. The second waveform may include a sustained intensity period during which the frequency of the second waveform is at the target frequency while the amplitude remains at the target amplitude.

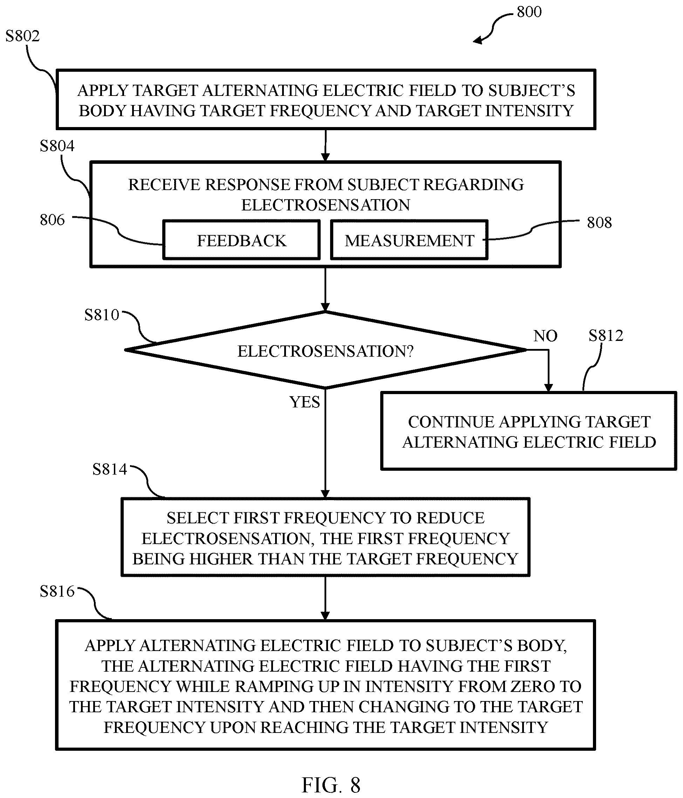

FIG. 8 depicts an example method 800 of delivering an alternating electric field to a subject's body. The method 800 includes, at step S802, applying a target alternating electric field to the subject's body to treat the subject's body. The target alternating electric field has a target frequency and a target intensity, the target frequency and the target intensity having a known efficacy for treating the subject's body. At step S804, the method 800 includes receiving a response from the subject regarding electrosensation. The response from the subject may include feedback 806 from the subject, a measurement 808 (e.g., sensor measurement) of the subject, or both. At step S810, the method 800 includes determining if the response indicates that the subject's body experiences electrosensation from the target alternating electric field. If at step S810 the response indicates that the subject's body does not experience electrosensation, the method 800 proceeds to step S812: continuing to apply the target alternating electric field to the subject's body to treat the subject's body.

If at step S810 the response indicates that the subject's body experiences electrosensation, the method 800 proceeds to step S814: selecting a first frequency for an alternating electric field to reduce the electrosensation experienced by the subject's body. The first frequency is higher than the target frequency. At step S816, the method 800 includes applying an alternating electric field to the subject's body, the alternating electric field having the first frequency while ramping up in intensity from zero to the target intensity and then changing to the target frequency upon reaching the target intensity. The method 800 of FIG. 8 allows for tuning the process of reducing or eliminating electrosensation to the individual subject, as not all subjects will experience electrosensation from a target alternating electric field.

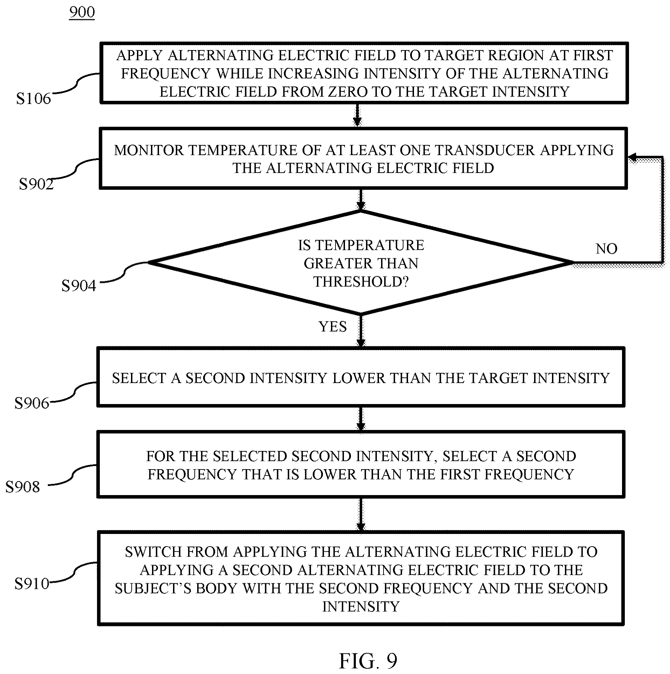

FIG. 9 depicts an example method 900 of delivering an alternating electric field to a subject's body as temperature rises. In method 900, the intensity and/or frequency of the alternating electric field may be modified as temperature changes. At step S902, after applying the alternating electric field with the first frequency while increasing an intensity of the alternating electric field from zero to the target intensity in step S106, the temperature where the alternating electric field is applied can be monitored. For example, the temperature of the transducer that applies the alternating electric field, the temperature of the target region, and/or the temperature of the skin of the subject at or near where the alternating electric field is applied can be monitored. A sensor to monitor the temperature may be part of or separate from the transducer.

At step S904, if the measured temperature is equal to or lower than a predetermined threshold, flow proceeds to step S902, and the loop is repeated. The alternating electric field with the first frequency and the first intensity may continue to be applied to the subject's body. In one example, the predetermined threshold temperature of the transducer may be approximately 37°C to approximately 39°C, or approximately 38.5°C to approximately 39°C, or approximately 37°C to approximately 41.5°C On the other hand, if the measured temperature is greater than a predetermined threshold, the flow proceeds to step S906.

At step S906, a second intensity can be selected. In non-limiting embodiments, the second intensity can be lower than the first intensity. For example, the second intensity may be lower than the target intensity by at least 10% than the first intensity (e.g., at least approximately 20% lower, 30% lower, 40% lower, 50% lower, or 60% lower). For example, the second intensity may be lower than the target intensity by at least 0.1 V/cm lower than the first intensity (e.g., at least approximately 0.2 V/cm, 0.3 V/cm, 0.4 V/cm, 0.5 V/cm, 0.6 V/cm, 0.7 V/cm, 0.8 V/cm, 0.9 V/cm, 1 V/cm, or 2 V/cm lower). For example, the second intensity can range from approximately 0.1 V/cm to approximately 20 V/cm, e.g., approximately 0.5 V/cm to approximately 10 V/cm.

At step S908, a second frequency can be selected after selecting the second intensity. In one example, the second frequency can be lower than the first frequency and closer to or at the target frequency in order to compensate for the decreased efficacy caused by lowering the intensity. For example, the second frequency can be lower than the first frequency by at least 10% (e.g., at least approximately 20%, 30%, 40%, 50%, or 60%) without going below the target frequency. In one example, the second frequency can be lower than the target frequency by at least approximately 10 kHz (e.g., at least approximately 20 kHz, 30 kHz, 50 kHz, 75 kHz, 100 kHz, 150 kHz, 200 kHz, or 300 kHz) without going below the target frequency. In one example, the second frequency can be based on the range of the first frequency.

At step S910, applying the alternating electric field with the first frequency and the first intensity to the subject's body may be switched to applying a second alternating electric field with the second frequency and the second intensity to the subject's body. In one example, the second alternating electric field can provide similar efficacy to the alternating electric field with the first frequency and the first intensity. In another example, the second alternating electric field can provide better efficacy than the alternating electric field with the first frequency and the first intensity. In non-limiting embodiments, the alternating electric fields can be generated from the same transducer or different transducers. In non-limiting embodiments, the first alternating electric field may be continuously switched to the second alternating electric field without turning off the system/transducer or a wait time.

### EXPERIMENTAL RESULTS

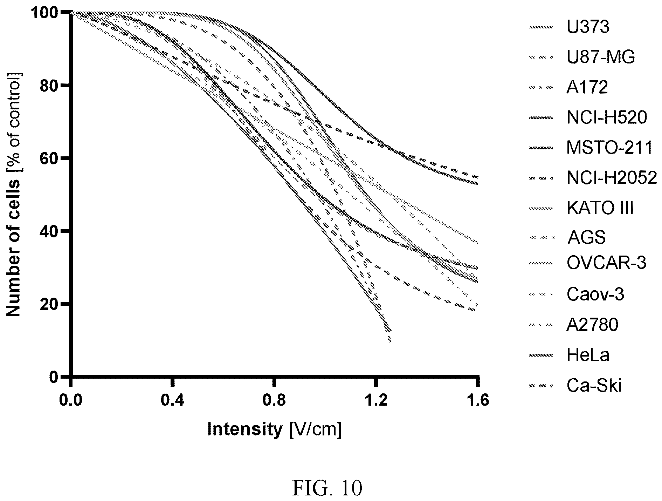

FIG. 10 depicts an example cytotoxicity analysis showing the effects of the field intensity on the survival of various types of cancer. The cytotoxicity analysis was performed by counting cell numbers (percent of control) following 72 hours of application of the disclosed frequency TTFields to cell lines from various types of cancers: glioblastoma (U373, U87-MG, and A172; 200 kHz), lung (NCI-H520 and A549; 150 kHz), mesothelioma (MSTO-211 and H2052; 150 kHz), gastric (KATO III and AGS; 150 kHz), ovarian (OVCAR-3, Caov-3, and A2780; 200 kHz), and cervical (HeLa and Ca-Ski; 150 kHz).

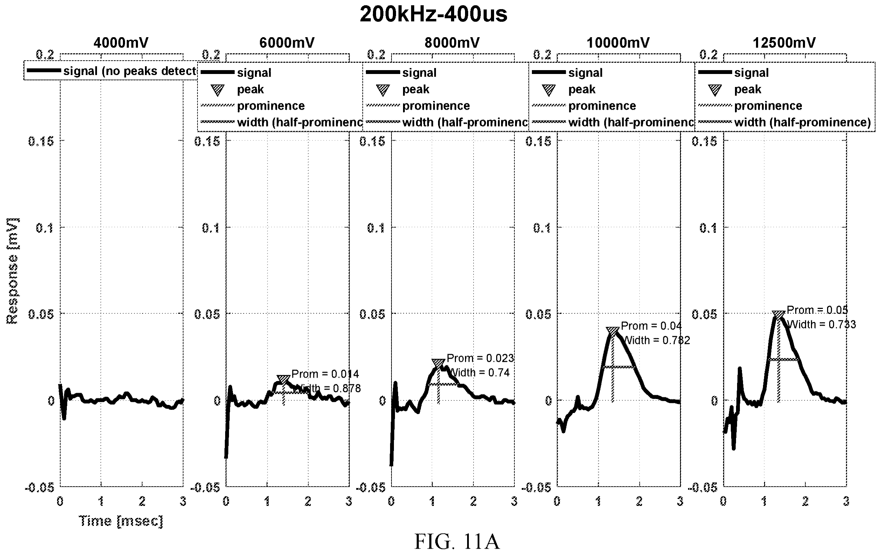

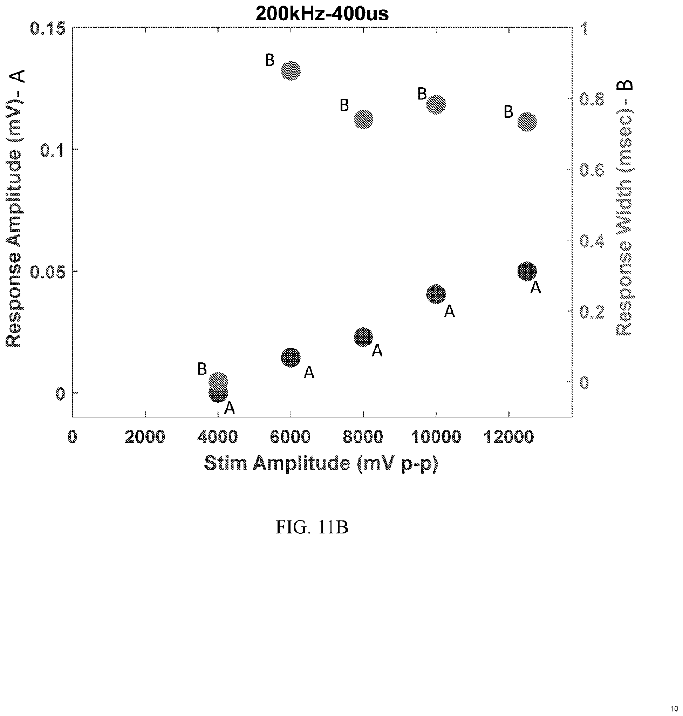

FIGS. 11A-11C depict experimental stimulation of a sciatic rat nerve bundle with alternating electric fields. FIG. 11A depicts five graphs showing the nerve response after treatment with an increasing voltage (4,000 mV, 6,000 mV, 8,000 mV, 10,000 mV, and 12,500 mV). As voltage was increased, the nerve response increased. FIG. 11B depicts a graph plotting the stimulation amplitude in mV p-p vs. the response amplitude in mV (blue circles, "A") and the response width in msec (red circles, "B"). The response width is a measurement proxy for velocity along the nerve bundle. As the response amplitude increased more nerves in the nerve bundle were activated.

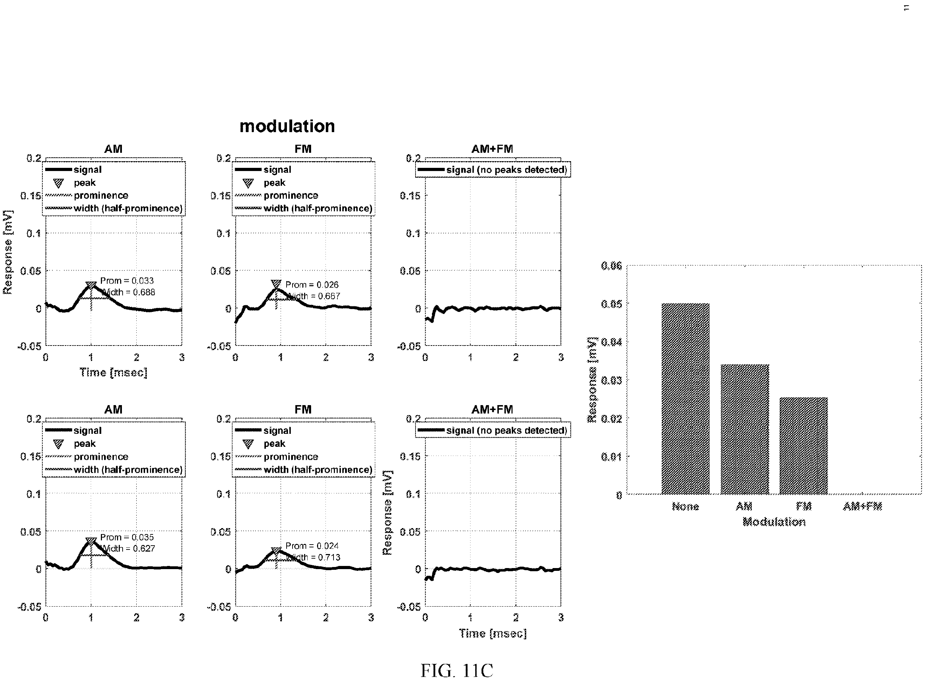

FIG. 11C depicts graphs representing an experiment where amplitude, frequency, and both amplitude and frequency were dimmed for 100 milliseconds. Human testing was conducted with a 100 ms dimming-up cycle on an OPTUNE ^{®} electric field generator, in which frequency was decreased from 300 Hz to 100 Hz. The threshold for electro-sensation was 0.8 A when amplitude was increased in 1 milli steps. The threshold for electro-sensation was about 1.4-1.5 A when both amplitude and frequency were dimmed.

The AM graph shows a nerve response to a stimulus in which amplitude increased from 500 mV to 12.5 volts in 100 microseconds. The FM graph shows a similar response to a stimulus in which the frequency was reduced from 400 Hz to 200 Hz in 100 microseconds. The AM + FM graph shows that there was no response to a stimulus in which the amplitude increased and the frequency was decreased simultaneously. The bottom three graphs depict a repetition of the experiment depicted in the top three graphs, which produced appreciably similar results. The right graph depicts the measured response for each treatment; increasing both amplitude and frequency produced no action potential.

The invention includes other illustrative embodiments ("Embodiments") as follows.

Embodiment 1: A method to treat a target region of a subject's body with an alternating electric field, the method comprising: applying the alternating electric field to the target region of the subject's body at a first frequency while increasing an intensity of the alternating electric field from zero to a target intensity; and changing the frequency of the alternating electric field from the first frequency to a target frequency while maintaining the alternating electric field at the target intensity, wherein the target frequency and the target intensity have a known efficacy for treating the target region of the subject's body with the alternating electric field; and wherein the first frequency is greater than the target frequency.

Embodiment 2: The method of Embodiment 1, wherein changing the frequency of the alternating electric field is performed instantly at one time during application of the alternating electric field.

Embodiment 3: The method of Embodiment 2, wherein: the alternating electric field is applied using a voltage generated according to an AC voltage waveform, and changing the frequency of the alternating electric field is performed at a time corresponding to a zero crossing of the AC voltage waveform.

Embodiment 4: The method of Embodiment 1, wherein changing the frequency of the alternating electric field is performed in less than a period of 200 ms.

Embodiment 5: The method of Embodiment 4, wherein: the alternating electric field is applied using a voltage generated according to an AC voltage waveform, and changing the frequency of the alternating electric field is performed at a time corresponding to a zero crossing of the AC voltage waveform.

Embodiment 6: The method of Embodiment 1, wherein changing the frequency of the alternating electric field is performed gradually over a period of time during application of the alternating electric field.

Embodiment 7: The method of Embodiment 6, wherein: the alternating electric field is applied using a voltage generated according to an AC voltage waveform, and changing the frequency of the alternating electric field is performed in a stepwise manner with each shift happening at a different zero crossing of the AC voltage waveform.

Embodiment 8: The method of claim 1, further comprising: after a first period of time, halting application of the alternating electric field to complete a first duty cycle of the alternating electric field; applying one or more subsequent duty cycles of the alternating electric field, each subsequent duty cycle following the same pattern of intensities and frequencies as the first duty cycle.

Embodiment 9: The method of Embodiment 8, wherein the alternating electric field is applied using a voltage generated according to an AC voltage waveform, with each duty cycle of the alternating electric field being applied according to the same AC voltage waveform.

Embodiment 10: The method of Embodiment 8, further comprising: after a second period of time, switching from applying the alternating electric field to the target region of the subject's body to applying a second alternating electric field to the target region of the subject's body with a second frequency that is lower than the first frequency and closer to or at the target frequency throughout each duty cycle.

Embodiment 11: The method of Embodiment 10, wherein the second period of time is approximately 12 hours to approximately 4 weeks.

Embodiment 12: The method of Embodiment 8, wherein the first period of time is less than three seconds.

Embodiment 13: The method of Embodiment 1, wherein the target frequency is approximately 50 kHz to approximately 1 MHz.

Embodiment 14: The method of Embodiment 1, wherein the target frequency is approximately 50 kHz to approximately 500 kHz.

Embodiment 15: The method of Embodiment 1, wherein the first frequency is at least approximately 10% higher than the target frequency.

Embodiment 16: The method of Embodiment 1, wherein the first frequency is at least approximately 10 kHz higher than the target frequency.

Embodiment 17: The method of Embodiment 1, wherein the target intensity is approximately 0.1 V/cm to approximately 10.0 V/cm.

Embodiment 18: The method of Embodiment 1, wherein the alternating electric field is configured to reduce electrosensation of the subject compared to when an alternating electric field is applied at the target frequency for its entire duty cycle.

Embodiment 19: The method of Embodiment 1, wherein the target region is glioblastoma (GBM), wherein the target frequency is approximately 100 kHz to approximately 300 kHz.

Embodiment 20: The method of Embodiment 1, wherein the target region is an endothelial or epithelial tight junction, wherein the target frequency is approximately 50 kHz to approximately 190 kHz.

Embodiment 21: The method of Embodiment 1, wherein the alternating electric field is delivered to the subject's body to change cancer cell membrane permeability, wherein the target frequency is approximately 100 kHz to approximately 500 kHz.

Embodiment 22: The method of Embodiment 1, wherein the alternating electric field is delivered to the subject's body via a pair of transducer arrays positioned on a head of the subject's body, wherein the target region is in the subject's brain.

Embodiment 23: The method of Embodiment 1, wherein the alternating electric field is delivered to the subject's body via a pair of transducer arrays positioned on at least one of a thorax, an abdomen, or one or both thighs of the subject's body, wherein the target region is in the subject's torso.

Embodiment 24: An apparatus for treating a target region of a subject's body with an alternating electric field, the apparatus comprising: a first pair of transducers configured to apply a first alternating electric field to a target region of the subject's body; a voltage generator coupled to the first pair of transducers and capable of providing a first voltage to the first pair of transducers for applying the first alternating electric field; and a controller coupled to the voltage generator, the controller comprising one or more processors and memory accessible by the one or more processors, the memory storing instructions that when executed by the one or more processors, cause the controller to instruct the voltage generator to generate the first voltage according to a first waveform having: an initial ramp-up period during which an amplitude of the first waveform increases from zero to a target amplitude while a frequency of the first waveform is at a first frequency; and a sustained intensity period during which the frequency of the first waveform changes to a target frequency lower than the first frequency while the amplitude of the first waveform remains at the target amplitude.

Embodiment 25: The apparatus of Embodiment 24, wherein the frequency of the first waveform changes instantly from the first frequency to the target frequency.

Embodiment 26: The apparatus of Embodiment 25, wherein the frequency of the first waveform changes at a zero crossing of the first waveform

Embodiment 27: The apparatus of Embodiment 24, wherein the frequency of the first waveform changes from the first frequency to the target frequency in less than 200 ms.

Embodiment 28: The apparatus of Embodiment 27, wherein the frequency of the first waveform changes at a zero crossing of the first waveform.

Embodiment 29: The apparatus of Embodiment 24, wherein the frequency of the first waveform changes gradually from the first frequency to the target frequency.

Embodiment 30: The apparatus of Embodiment 29, wherein the frequency of the first waveform changes in a stepwise manner with each shift happening at a different zero crossing of the first waveform.

Embodiment 31: The apparatus of Embodiment 24, wherein the voltage generator is coupled to the first pair of transducers and capable of providing a third voltage to the first pair of transducers for applying a third alternating electric field to a target region of the subject's body, and the instructions, when executed by the one or more processors, cause the controller to instruct the voltage generator to generate the third voltage according to a second waveform having: an initial ramp-up period during which an amplitude of the second waveform increases from zero to the target amplitude while a frequency of the second waveform is at a frequency lower than the first frequency; and a sustained intensity period during which the frequency of the second waveform is at the target frequency while the amplitude of the second waveform remains at the target amplitude.

Embodiment 32: The apparatus of Embodiment 31, wherein the second waveform is at the target frequency during the initial ramp-up period.

Embodiment 33: The apparatus of Embodiment 24, further comprising: a second pair of transducers configured to apply a second alternating electric field to a target region of the subject's body, wherein the voltage generator is coupled to the second pair of transducers and capable of providing a second voltage to the second pair of transducers for applying the second alternating electric field, and the instructions, when executed by the one or more processors, cause the controller to instruct the voltage generator to generate the second voltage according to the first waveform.

Embodiment 34: The apparatus of Embodiment 33, wherein the instructions, when executed by the one or more processors, cause the controller to instruct the voltage generator to alternately switch between providing the first voltage to the first pair of transducers and providing the second voltage to the second pair of transducers at regular intervals.

Embodiment 35: A method to deliver an alternating electric field to a subject's body, the method comprising: applying a target alternating electric field to the subject's body to treat the subject's body, the target alternating electric field having a target frequency and a target intensity, the target frequency and the target intensity having a known efficacy for treating the subject's body; receiving a response from the subject regarding electrosensation, wherein the response from the subject comprises at least one of feedback from the subject or a measurement of the subject; if the response indicates that the subject's body experiences electrosensation from the target alternating electric field, selecting a first frequency for an alternating electric field to reduce the electrosensation experienced by the subject's body from the target alternating electric field, the first frequency being higher than the target frequency; and applying an alternating electric field to the subject's body, the alternating electric field having the first frequency while ramping up in intensity from zero to the target intensity and then changing to the target frequency upon reaching the target intensity.

Embodiment 36: An apparatus for treating a target region of a subject's body with an alternating electric field, the apparatus comprising: a pair of transducers configured to apply a first alternating electric field to a target region of the subject's body; and a controller implemented with at least one processor to provide signals to the pair of transducers for controlling the first alternating electric field such that: the first alternating electric field begins at a first frequency; the first alternating electric field increases from zero intensity to a target intensity while remaining at the first frequency; and the first alternating electric field changes from the first frequency to a target frequency after reaching the target intensity, the target frequency being lower than the first frequency.

Embodiment 37: A method to treat a target region of a subject's body with an alternating electric field, the method comprising: applying the alternating electric field to the target region of the subject's body at a first frequency while increasing a current of the alternating electric field from zero to a target current; and changing the frequency of the alternating electric field from the first frequency to a target frequency while maintaining the alternating electric field at the target current, wherein the target frequency and the target current have a known efficacy for treating the target region of the subject's body with the alternating electric field; and wherein the first frequency is greater than the target frequency.

Illustrative Embodiment 38. A method to deliver an alternating electric field to a subject's body, the method comprising: applying a target alternating electric field to the subject's body to treat the subject's body, the target alternating electric field having a target frequency and a target intensity, the target frequency and the target intensity having a known efficacy for treating the subject's body; receiving feedback regarding electrosensation experienced by the subject's body from applying the target alternating electric field; if the subject's body experiences electrosensation from the target alternating electric field at the target frequency and the target intensity, select at least one of a first frequency or a first intensity for an alternating electric field to reduce the electrosensation experienced by the subject's body from the target alternating electric field, the first frequency being higher than the target frequency, the first intensity being lower than the target intensity; and applying a first alternating electric field to the subject's body to treat the subject's body, the first alternating electric field having at least one of the first frequency or the first intensity.

Illustrative Embodiment 39. The method of Illustrative Embodiment 38, wherein the first frequency is at least approximately 10% higher than the target frequency and wherein the first intensity is at least approximately 10% lower than the target intensity.

Illustrative Embodiment 40. The method of Illustrative Embodiment 39, further comprising: after an interval of time, ceasing to apply the first alternating electric field to the subject's body; applying a second alternating electric field to the subject's body with at least one of a second frequency closer to or at the target frequency or a second intensity closer to or at the target intensity.

Illustrative Embodiment 41. A method to deliver an alternating electric field to a subject's body, the method comprising: applying the alternating electric field to a target region of the subject's body, wherein the alternating electric field has a first frequency and a first intensity, wherein for a target frequency and a target intensity of the alternating electric field, the subject experiences electrosensation, wherein the first intensity is the target intensity, and wherein the first frequency is greater than the target frequency.

Illustrative Embodiment 42. The method of Illustrative Embodiment 41, wherein the alternating electric field is configured to reduce electrosensation of the subject compared to when an alternating electric field is applied at the target frequency and the target intensity.

Illustrative Embodiment 43. The method of Illustrative Embodiment 41, the method further comprises prior to applying the alternating electric field, determining the first intensity of the alternating electric field; and after the first intensity is determined, identifying the first frequency of the alternating electric field based on a response from the subject regarding electrosensation experienced by the subject, wherein the first frequency is a frequency of the alternating electric field that reduces the subject's electrosensation with the first intensity.

Illustrative Embodiment 44. The method of Illustrative Embodiment 43, wherein identifying the first frequency of the alternating electric field comprises: decreasing a frequency level of the alternating electric field stepwise from an initial frequency until the response from the subject indicates that the subject experiences the electrosensation.

Illustrative Embodiment 45. The method of Illustrative Embodiment 43, wherein the response from the subject comprises at least one of feedback from the subject or a measurement of the subject.

Illustrative Embodiment 46. The method of Illustrative Embodiment 41, wherein the target frequency is approximately 100 kHz to approximately 400 kHz, and the target intensity is approximately 0.5 V/cm to approximately 10.0 V/cm.

Illustrative Embodiment 47. The method of Illustrative Embodiment 41, the method further comprises when a temperature where the alternating electric field is applied reaches a threshold temperature, determining a second intensity for the alternating electric field, wherein the second intensity is lower than the first intensity; identifying a second frequency of the alternating electric field, wherein the second frequency is lower than the first frequency; switching from applying the alternating electric field to applying another alternating electric field with the second intensity and the second frequency.

Illustrative Embodiment 48. The method of Illustrative Embodiment 41, the method further comprises after an interval of time, switching from applying the alternating electric field to the target region of the subject's body to applying a second alternating electric field to the target region of the subject's body with a lower frequency closer to or at the target frequency.

Illustrative Embodiment 49. The method of Illustrative Embodiment 48, wherein the interval between applying the alternating electric field with the first frequency and the second alternating electric field with the lower frequency is approximately 12 hours to approximately 4 weeks (e.g., approximately 12 hours to approximately 2 weeks).

Illustrative Embodiment 50. The method of Illustrative Embodiment 41, wherein the first frequency is at least approximately 10% higher than the target frequency.

Illustrative Embodiment 51. The method of Illustrative Embodiment 41, wherein the first frequency is at least approximately 10 kHz higher than the target frequency.

Illustrative Embodiment 52. The method of Illustrative Embodiment 41, wherein the target region is glioblastoma (GBM), wherein the target frequency is approximately 50 kHZ to approximately 350 kHz, and the target intensity is approximately 1 V/cm to approximately 5 V/cm.

Illustrative Embodiment 53. The method of Illustrative Embodiment 41, wherein the target region is a blood-brain barrier (BBB), wherein the target frequency is approximately 50 kHz to approximately 190 kHz, and the target intensity is approximately 1 V/cm to approximately 5 V/cm.

Illustrative Embodiment 54. The method of Illustrative Embodiment 41, wherein the alternating electric field is delivered to the subject's body to change cell membrane permeability, wherein the target frequency is approximately 50 kHz to approximately 550 kHz, and the target intensity is approximately 0.5 V/cm to approximately 20 V/cm.

Illustrative Embodiment 55. An apparatus for treating a target region of a subject's body with an alternating electric field, the apparatus comprising: a transducer configured to apply the alternating electric field to a target region of the subject's body; and a controller implemented with at least one processor to provide signals to the transducer for the alternating electric field, wherein the alternating electric field has a first frequency and a target intensity, wherein for a frequency and an intensity having a known efficacy for treating the target region of the subject's body with the alternating electric field, the subject experiences electrosensation, wherein the first intensity is the intensity having the known efficacy, and wherein the first frequency is greater than the frequency having the known efficacy.

Illustrative Embodiment 56. The apparatus of Illustrative Embodiment 55, wherein the controller comprises one or more processors; and memory storing processor-executable instructions that, when executed by the one or more processors, cause the controller to determine the first intensity of the alternating electric field prior to applying the alternating electric field; and after the first intensity is determined, identify the first frequency of the alternating electric field based on a response from the subject regarding electrosensation experienced by the subject, wherein the first frequency is a frequency of the alternating electric field that reduces the subject's electrosensation with the first intensity.

Illustrative Embodiment 57. The apparatus of Illustrative Embodiment 56, wherein the response from the subject comprises at least one of feedback from the subject or a measurement of the subject.

Illustrative Embodiment 58. The apparatus of Illustrative Embodiment 56, wherein the memory storing processor-executable instructions that, when executed by the one or more processors, further cause the apparatus to decrease a frequency level of the alternating electric field stepwise from an initial frequency until feedback from the subject indicates that subject experiences electrosensation.

Illustrative Embodiment 59. The apparatus of Illustrative Embodiment 56, wherein the memory storing processor-executable instructions that, when executed by the one or more processors, further cause the apparatus to decrease a frequency level of the alternating electric field stepwise from an initial frequency until a measurement of nerve stimulation of the subject indicates that subject experiences electrosensation.

Illustrative Embodiment 60. The apparatus of Illustrative Embodiment 55, wherein the controller comprises one or more processors; and memory storing processor-executable instructions that, when executed by the one or more processors, cause the controller to when a temperature where the alternating electric field is applied reaches a threshold temperature, determine a second intensity of the alternating electric field, the second intensity being lower than the first intensity; identifying a second frequency of the alternating electric field, wherein the second frequency is lower than the first frequency; and apply the alternating electric field with the second intensity and the second frequency.

Illustrative Embodiment 61. The apparatus of Illustrative Embodiment 55, wherein the alternating electric field is capable of applying a tumor treating field, opening a blood-brain barrier of the subject, disrupting a cell membrane of the subject, or changing permeability of a cell membrane of the subject.

Illustrative Embodiment 62. An apparatus to calibrate delivery of an alternating electric field to a subject's body, comprising: one or more processors; and memory storing processor-executable instructions that, when executed by the one or more processors, cause the apparatus to determine a target frequency and a target intensity for application of the alternating electric field to treat a target region of the subject's body, the target frequency and the target intensity having known efficacy to treat the subject's body; and select a first frequency for application of an alternating electric field to treat the target region of the subject's body, the first frequency selected to reduce electrosensation in the subject's body experienced at the target intensity, wherein the first frequency is higher than the target frequency.

Illustrative Embodiment 63. The apparatus of Illustrative Embodiment 62, wherein the target frequency and the target intensity have known efficacy for treating a tumor, opening a blood-brain barrier of the subject, disrupting a cell membrane of the subject, or changing permeability of a cell membrane of the subject.

Illustrative Embodiment 64. A method to deliver an alternating electric field to a subject's body, the method comprising: applying a target alternating electric field to the subject's body to treat the subject's body, the target alternating electric field having a target frequency and a target intensity, the target frequency and the target intensity having a known efficacy for treating the subject's body; receiving a response from the subject regarding electrosensation, wherein the response from the subject comprises at least one of feedback from the subject or a measurement of the subject; if the response indicates that subject's body experiences electrosensation from the target alternating electric field at the target frequency and the target intensity, selecting a first frequency for an alternating electric field to reduce the electrosensation experienced by the subject's body from the target alternating electric field, the first frequency being higher than the target frequency; and applying a first alternating electric field to the subject's body to treat the subject's body, the first alternating electric field having the first frequency and the target intensity.

Illustrative Embodiment 65. The method of Illustrative Embodiment 64, wherein the first frequency is at least approximately 10% higher than the target frequency.

Illustrative Embodiment 66. The method of Illustrative Embodiment 64, the method further comprises after an interval of time, switching from applying the first alternating electric field to the subject's body to applying a second alternating electric field to the subject's body with a second frequency closer to or at the target frequency.

Illustrative Embodiment 67: A method to treat a target region of a subject's body with an alternating electric field, the method comprising: applying the alternating electric field to the target region of the subject's body at a frequency and an intensity, wherein the frequency is a first frequency, wherein the intensity is a first intensity; and increasing the intensity from the first intensity to a target intensity while decreasing the frequency from the first frequency to a target frequency, wherein the target frequency and the target intensity have a known efficacy for treating the target region of the subject's body with the alternating electric field; wherein the first frequency is greater than the target frequency; and wherein the first intensity is lesser than the target intensity.

Illustrative Embodiment 68: The method of Embodiment 67, wherein the frequency is decreased from the first frequency to the target frequency instantly at one time during application of the alternating electric field.

Illustrative Embodiment 69: The method of Embodiment 67, wherein increasing the intensity while decreasing the first frequency of the alternating electric field is performed in less than a period of 200 ms.

Illustrative Embodiment 70: The method of Embodiment 67, wherein increasing the intensity while decreasing the first frequency of the alternating electric field is performed gradually over a period of time during application of the alternating electric field.

Illustrative Embodiment 71: The method of claim 67, further comprising: after a first period of time, halting application of the alternating electric field to complete a first duty cycle of the alternating electric field; applying one or more subsequent duty cycles of the alternating electric field, each subsequent duty cycle following the same pattern of intensities and frequencies as the first duty cycle.

Illustrative Embodiment 72: The method of Embodiment 71, wherein the alternating electric field is applied using a voltage generated according to an AC voltage waveform, with each duty cycle of the alternating electric field being applied according to the same AC voltage waveform.

Illustrative Embodiment 73: The method of Embodiment 71, further comprising: after a second period of time, switching from applying the alternating electric field to the target region of the subject's body to applying a second alternating electric field to the target region of the subject's body with a second frequency that is lower than the first frequency and closer to or at the target frequency throughout each duty cycle.

Illustrative Embodiment 74: The method of Embodiment 73, wherein the second period of time is approximately 12 hours to approximately 4 weeks.

Illustrative Embodiment 75: The method of Embodiment 71, wherein the first period of time is less than three seconds.

Illustrative Embodiment 76: The method of Embodiment 67, wherein the target frequency is approximately 50 kHz to approximately 1 MHz.

Illustrative Embodiment 77: The method of Embodiment 67, wherein the target frequency is approximately 50 kHz to approximately 500 kHz.

Illustrative Embodiment 78: The method of Embodiment 67, wherein the first frequency is at least approximately 10% higher than the target frequency.

Illustrative Embodiment 79: The method of Embodiment 67, wherein the first frequency is at least approximately 10 kHz higher than the target frequency.

Illustrative Embodiment 80: The method of Embodiment 67, wherein the target intensity is approximately 0.1 V/cm to approximately 10.0 V/cm.

Illustrative Embodiment 81: The method of Embodiment 67, wherein the alternating electric field is configured to reduce electrosensation of the subject compared to when an alternating electric field is applied at the target frequency for its entire duty cycle.

Illustrative Embodiment 82: The method of Embodiment 67, wherein the target region is glioblastoma (GBM), wherein the target frequency is approximately 100 kHz to approximately 300 kHz.

Illustrative Embodiment 83: The method of Embodiment 67, wherein the target region is an endothelial or epithelial tight junction, wherein the target frequency is approximately 50 kHz to approximately 190 kHz.

Illustrative Embodiment 84: The method of Embodiment 67, wherein the alternating electric field is delivered to the subject's body to change cancer cell membrane permeability, wherein the target frequency is approximately 100 kHz to approximately 500 kHz.

Illustrative Embodiment 85: The method of Embodiment 67, wherein the alternating electric field is delivered to the subject's body via a pair of transducer arrays positioned on a head of the subject's body, wherein the target region is in the subject's brain.

Illustrative Embodiment 86: The method of Embodiment 67, wherein the alternating electric field is delivered to the subject's body via a pair of transducer arrays positioned on at least one of a thorax, an abdomen, or one or both thighs of the subject's body, wherein the target region is in the subject's torso.

Illustrative Embodiment 87: An apparatus for treating a target region of a subject's body with an alternating electric field, the apparatus comprising: a first pair of transducers configured to apply a first alternating electric field to a target region of the subject's body; a voltage generator coupled to the first pair of transducers and capable of providing a first voltage to the first pair of transducers for applying the first alternating electric field; and a controller coupled to the voltage generator, the controller comprising one or more processors and memory accessible by the one or more processors, the memory storing instructions that when executed by the one or more processors, cause the controller to perform operations comprising: applying the alternating electric field to the target region of the subject's body at a first frequency while increasing an intensity of the alternating electric field from zero to a target intensity; and changing the frequency of the alternating electric field from the first frequency to a target frequency while maintaining the alternating electric field at the target intensity, wherein the target frequency and the target intensity have a known efficacy for treating the target region of the subject's body with the alternating electric field; and wherein the first frequency is greater than the target frequency.

Illustrative Embodiment 88: An apparatus for treating a target region of a subject's body with an alternating electric field, the apparatus comprising: a first pair of transducers configured to apply a first alternating electric field to a target region of the subject's body; a voltage generator coupled to the first pair of transducers and capable of providing a first voltage to the first pair of transducers for applying the first alternating electric field; and a controller coupled to the voltage generator, the controller comprising one or more processors and memory accessible by the one or more processors, the memory storing instructions that when executed by the one or more processors, cause the controller to perform operations comprising: applying the alternating electric field to the target region of the subject's body at a frequency and an intensity, wherein the frequency is a first frequency, wherein the intensity is a first intensity; and increasing the intensity from the first intensity to a target intensity while decreasing the frequency from the first frequency to a target frequency, wherein the target frequency and the target intensity have a known efficacy for treating the target region of the subject's body with the alternating electric field, wherein the first frequency is greater than the target frequency, and wherein the first intensity is lesser than the target intensity.

Embodiments illustrated under any heading or in any portion of the disclosure may be combined with embodiments illustrated under the same or any other heading or other portion of the disclosure unless otherwise indicated herein or otherwise clearly contradicted by context.

Numerous modifications, alterations, and changes to the described embodiments are possible without departing from the scope of the present invention defined in the claims. It is intended that the present invention not be limited to the described embodiments, but that it has the full scope defined by the language of the following claims, and equivalents thereof.

### Embodiments:

1. An apparatus for treating a target region of a subject's body with an alternating electric field, the apparatus comprising:
   a first pair of transducers configured to apply a first alternating electric field to a target region of the subject's body;
   a voltage generator coupled to the first pair of transducers and capable of providing a first voltage to the first pair of transducers for applying the first alternating electric field; and
   a controller coupled to the voltage generator, the controller comprising one or more processors and memory accessible by the one or more processors, the memory storing instructions that when executed by the one or more processors, cause the controller to perform operations comprising:
      applying the alternating electric field to the target region of the subject's body at a first frequency while increasing an intensity of the alternating electric field from zero to a target intensity; and
      changing the frequency of the alternating electric field from the first frequency to a target frequency while maintaining the alternating electric field at the target intensity,
      wherein the target frequency and the target intensity have a known efficacy for treating the target region of the subject's body with the alternating electric field; and
      wherein the first frequency is greater than the target frequency.
2. The apparatus of embodiment 1, wherein:
   the alternating electric field is applied using a voltage generated according to an AC voltage waveform, and
   changing the frequency of the alternating electric field is performed at a time corresponding to a zero crossing of the AC voltage waveform.
3. The apparatus of embodiment 1, wherein changing the frequency of the alternating electric field is performed gradually over a period of time during application of the alternating electric field.
4. The apparatus of embodiment 3, wherein:
   the alternating electric field is applied using a voltage generated according to an AC voltage waveform, and
   changing the frequency of the alternating electric field is performed in a stepwise manner with each shift happening at a different zero crossing of the AC voltage waveform.
5. The apparatus of embodiment 1, further comprising:
   after a first period of time, halting application of the alternating electric field to complete a first duty cycle of the alternating electric field;
   applying one or more subsequent duty cycles of the alternating electric field, each subsequent duty cycle following a similar pattern of intensities and frequencies as the first duty cycle.
6. An apparatus for treating a target region of a subject's body with an alternating electric field, the apparatus comprising:
   a first pair of transducers configured to apply a first alternating electric field to a target region of the subject's body;
   a voltage generator coupled to the first pair of transducers and capable of providing a first voltage to the first pair of transducers for applying the first alternating electric field; and
   a controller coupled to the voltage generator, the controller comprising one or more processors and memory accessible by the one or more processors, the memory storing instructions that when executed by the one or more processors, cause the controller to instruct the voltage generator to generate the first voltage according to a first waveform having:
      an initial ramp-up period during which an amplitude of the first waveform increases from zero to a target amplitude while a frequency of the first waveform is at a first frequency; and
      a sustained intensity period during which the frequency of the first waveform changes to a target frequency lower than the first frequency while the amplitude of the first waveform remains at the target amplitude.
7. The apparatus of embodiment 6, wherein the frequency of the first waveform changes instantly from the first frequency to the target frequency.
8. The apparatus of embodiment 7, wherein the frequency of the first waveform changes at a zero crossing of the first waveform.
9. The apparatus of embodiment 6, wherein the frequency of the first waveform changes from the first frequency to the target frequency in less than 200 ms.
10. The apparatus of embodiment 9, wherein the frequency of the first waveform changes at a zero crossing of the first waveform.
11. The apparatus of embodiment 6, wherein the frequency of the first waveform changes gradually from the first frequency to the target frequency.
12. The apparatus of embodiment 11, wherein the frequency of the first waveform changes in a stepwise manner with each shift happening at a different zero crossing of the first waveform.
13. The apparatus of embodiment 6, wherein the target frequency is approximately 50 kHz to approximately 500 kHz, and wherein the target intensity is approximately 0.1 V/cm to approximately 10.0 V/cm.
14. The apparatus of embodiment 6, wherein the first frequency is at least approximately 10 kHz higher than the target frequency.
15. An apparatus for treating a target region of a subject's body with an alternating electric field, the apparatus comprising:
   a first pair of transducers configured to apply a first alternating electric field to a target region of the subject's body;
   a voltage generator coupled to the first pair of transducers and capable of providing a first voltage to the first pair of transducers for applying the first alternating electric field; and
   a controller coupled to the voltage generator, the controller comprising one or more processors and memory accessible by the one or more processors, the memory storing instructions that when executed by the one or more processors, cause the controller to perform operations comprising:
      applying the alternating electric field to the target region of the subject's body at a frequency and an intensity, wherein the frequency is a first frequency, wherein the intensity is a first intensity; and
      increasing the intensity from the first intensity to a target intensity while decreasing the frequency from the first frequency to a target frequency,
      wherein the target frequency and the target intensity have a known efficacy for treating the target region of the subject's body with the alternating electric field,
      wherein the first frequency is greater than the target frequency, and
      wherein the first intensity is lesser than the target intensity.

## Claims

1. An apparatus for treating a target region of a subject's body with an alternating electric field, the apparatus comprising:
a first pair of transducers configured to apply a first alternating electric field to a target region of the subject's body;
a voltage generator coupled to the first pair of transducers and capable of providing a first voltage to the first pair of transducers for applying the first alternating electric field; and
a controller coupled to the voltage generator, the controller comprising one or more processors and memory accessible by the one or more processors, the memory storing instructions that when executed by the one or more processors, cause the controller to perform operations comprising:
applying the alternating electric field to the target region of the subject's body at a frequency and an intensity, wherein the frequency is a first frequency, wherein the intensity is a first intensity; and
increasing the intensity from the first intensity to a target intensity while decreasing the frequency from the first frequency to a target frequency,
wherein the target frequency and the target intensity have a known efficacy for treating the target region of the subject's body with the alternating electric field,
wherein the first frequency is greater than the target frequency, and
wherein the first intensity is lesser than the target intensity.

2. The apparatus of claim 1, wherein the first intensity is at least 10% lower than the target intensity,
optionally wherein the first intensity is at least 50% lower than the target intensity,
further optionally wherein the first intensity is at least 75% lower than the target intensity.

3. The apparatus of claim 1 or claim 2, wherein the change in frequency is gradual.

4. The apparatus of claim 3, wherein the change in frequency takes at least 0.1 ms,
optionally, wherein the change in frequency takes at least 5 ms,
further optionally, wherein the change in frequency takes at least 100 ms.

5. The apparatus of any preceding claim, wherein the change in intensity is gradual.

6. The apparatus of claim 5, wherein the change in intensity takes at least 0.1 ms,
optionally, wherein the change in intensity takes at least 5 ms,
further optionally, wherein the change in intensity takes at least 100 ms.

7. The apparatus of any preceding claim, wherein the increase in intensity and decrease in frequency do not start and stop at the same time.

8. The apparatus of any preceding claim, wherein the target frequency is between 50 kHz and 500 kHz.

9. The apparatus of any preceding claim, wherein the first frequency is at least 10% higher than the target frequency.

10. The apparatus of claim 9, wherein the first frequency is at least 50% higher than the frequency intensity.

11. The apparatus of claim 10, wherein the first frequency is at least 100% higher than the target intensity.

12. The apparatus of any preceding claim, wherein the first frequency is at least 10 kHz higher than the target frequency.

13. The apparatus of claim 12, wherein the first frequency is at least 50 kHz higher than the target frequency.

14. The apparatus of claim 13, wherein the first frequency is at least 100 kHz higher than the target frequency.

15. The apparatus of any preceding claim, wherein the first frequency is between 200 kHz and 600kHz.
